(19) Europäisches Patentamt / European Patent Office / Office européen des brevets

(11) **EP 3 182 884 B1**

(12) **FASCICULE DE BREVET EUROPEEN**

(45) Date de publication et mention de la délivrance du brevet:
**23.05.2018 Bulletin 2018/21**

(21) Numéro de dépôt: **15766062.2**

(22) Date de dépôt: **24.08.2015**

(51) Int Cl.:
**A61B 5/00** *(2006.01)*  **A61B 5/04** *(2006.01)*

(86) Numéro de dépôt international:
**PCT/EP2015/069330**

(87) Numéro de publication internationale:
**WO 2016/026980 (25.02.2016 Gazette 2016/08)**

(54) **MÉTHODE D'IMAGERIE MEG ASSISTÉE PAR IRM DE DIFFUSION**

DURCH DIFFUSIONS-MRT UNTERSTÜTZTES VERFAHREN ZUR MEG-BILDGEBUNG

MEG IMAGING METHOD ASSISTED BY DIFFUSION MRI

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.08.2014 FR 1457945**

(43) Date de publication de la demande:
**28.06.2017 Bulletin 2017/26**

(73) Titulaire: **Commissariat à l'Énergie Atomique et aux Énergies Alternatives 75015 Paris (FR)**

(72) Inventeurs:
• **LABYT, Etienne**
  **F-38950 Saint Martin Le Vinoux (FR)**
• **DURAND, Pierre**
  **38700 Corenc (FR)**

(74) Mandataire: **Brevalex**
  **56, Boulevard de l'Embouchure**
  **B.P. 27519**
  **31075 Toulouse Cedex 2 (FR)**

(56) Documents cités:
• **HAMID LAITH ET AL: "MEG-EEG fusion by Kalman filtering within a source analysis framework", THE EFFECT OF APPLIED COMPRESSIVE LOADING ON TISSUE-ENGINEERED CARTILAGE CONSTRUCTS CULTURED WITH TGF-BETA3, IEEE, 3 juillet 2013 (2013-07-03), pages 4819-4822, XP032489212, ISSN: 1557-170X, DOI: 10.1109/EMBC.2013.6610626 [extrait le 2013-09-25]**
• **BIN HE ET AL: "Multimodal Functional Neuroimaging: Integrating Functional MRI and EEG/MEG", IEEE REVIEWS IN BIOMEDICAL ENGINEERING, IEEE, USA, vol. 1, 1 janvier 2008 (2008-01-01), pages 23-40, XP011491243, ISSN: 1937-3333, DOI: 10.1109/RBME.2008.2008233**
• **LONG C J ET AL: "Large Scale Kalman Filtering Solutions to the Electrophysiological Source Localization Problem- A MEG Case Study", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 30 août 2006 (2006-08-30), pages 4532-4535, XP031339695, ISBN: 978-1-4244-0032-4**

**EP 3 182 884 B1**

**Description**

**DOMAINE TECHNIQUE**

La présente invention concerne de manière générale le domaine de l'imagerie et plus particulièrement de l'imagerie du tissu cérébral.

La méthode d'imagerie classique du tissu cérébral est celle par résonance magnétique nucléaire ou IRM. On rappelle que la résonance magnétique nucléaire exploite le fait que certains atomes, comme l'hydrogène, possèdent un moment magnétique de spin. Sur application d'un champ magnétique statique de forte intensité, les spins atomiques sont animés d'un mouvement rapide de précession (précession de Larmor), autour de la direction du champ magnétique, dénommée conventionnellement direction longitudinale.

Si l'on excite les atomes avec une onde électromagnétique radiofréquence à une fréquence prédéterminée, dite fréquence de Larmor, les moments magnétiques s'écartent progressivement de la direction longitudinale pour se placer dans le plan orthogonal à cette direction. L'application d'un champ magnétique statique uniforme ne permet pas d'obtenir une résolution spatiale du signal. Aussi superpose-t-on à ce champ uniforme un champ magnétique directionnel présentant un gradient dans une direction prédéterminée. Ce gradient code spatialement la fréquence de Larmor.

Lorsque l'on interrompt l'onde radiofréquence, les moments magnétiques qui se sont écartés de la direction longitudinale, reviennent progressivement s'aligner dans cette direction, avec un temps de relaxation dit temps de relaxation longitudinale, noté T1. De même, après excitation par l'onde RF, les moments magnétiques d'un même isotope situé dans un même champ statique doivent en théorie tourner de manière cohérente autour de l'axe longitudinal, autrement dit tourner de manière synchrone autour de cet axe, en conservant des différences de phase constantes entre eux. Après l'excitation, l'agitation moléculaire conduit à une décohérence de précession des différents moments et à une diminution du signal de réponse. Le temps caractéristique de la décohérence est encore dénommé temps de relaxation transversale, noté T2. Les temps de relaxation T1 et T2 dépendent du tissu que l'on analyse.

En modifiant les paramètres d'acquisition IRM, notamment la période de répétition des impulsions du signal RF d'excitation et le retard entre l'impulsion d'excitation et la fenêtre temporelle dans laquelle on analyse l'écho, on peut discriminer des tissus ayant des temps de relaxation T1 ou T2 différents. En particulier, si l'on utilise une période de répétition courte avec un retard court, on obtient une image pondérée en T1. Cette image est également appelée image IRM anatomique. En particulier une image IRM pondérée en T1 du cerveau permet de distinguer la substance blanche (axones) de la substance grise (cortex cérébral).

L'imagerie IRM a fait l'objet de nombreuses évolutions ces dernières décennies. L'une d'entre elles est l'imagerie IRM de diffusion permettant de représenter les caractéristiques locales de diffusion moléculaire de différentes espèces chimiques et principalement celles de l'eau. On pourra trouver une introduction à l'imagerie IRM de diffusion connue sous l'acronyme IRMD ou, selon la terminologie anglo-saxonne, DMRI *(Diffusion Magnetic Resonance Imaging)* dans l'article de S. Mori et al. intitulé « Diffusion magnetic resonance imaging: its principle and applications » publié dans la revue The Anatomical Record, No. 257, pp. 102-109, 1999. En bref, cette technique consiste à ne plus additionner un seul champ magnétique directionnel au champ magnétique uniforme mais successivement deux impulsions de champ magnétique directionnel de même direction mais de gradients opposés. Le premier gradient de champ est appelé gradient de déphasage (car il crée une dispersion de phase des moments dans le mouvement de précession) et le second gradient de champ est appelé gradient de « rephasage » ou de « refocalisation » (car il remet en phase les moments dans le mouvement de précession). En choisissant convenablement le temps séparant l'application des deux impulsions de champ magnétique directionnel on peut analyser la diffusion des espèces dans la direction de ce champ et en déduire la constante de diffusion dans cette direction. De manière générale, la IRMD permet de mesurer en chaque point (plus précisément pour chaque voxel) le tenseur de diffusion local représenté par la matrice :

$$\mathbf{D} = \begin{pmatrix} D_{xx} & D_{xy} & D_{xz} \\ D_{yx} & D_{yy} & D_{yz} \\ D_{zx} & D_{zy} & D_{zz} \end{pmatrix} \qquad (1)$$

Cette matrice est symétrique et donc diagonalisable à l'aide d'une matrice de passage orthogonale.

En un point donné, le tenseur de diffusion peut alors être représenté par la surface d'un ellipsoïde dont les axes propres sont définis par les vecteurs propres (orthogonaux deux à deux) de la matrice **D.** Lorsque le milieu est isotrope, les trois valeurs propres de la matrice sont égales à la constante de diffusion D dans le milieu, telle que définie dans la loi de Fick.

En règle générale, le milieu n'est pas isotrope et l'on peut exprimer la diffusivité moyenne du milieu par la moyenne, $\overline{\lambda}$, des valeurs propres de la matrice de diffusion :

$$\overline{\lambda} = Tr(\mathbf{D})/3 \qquad\qquad (2)$$

où $Tr(.)$ est la fonction trace. L'imagerie de diffusivité moyenne permet d'obtenir des informations très utiles sur le tissu cérébral. En particulier, elle permet d'identifier et de localiser un accident vasculaire cérébral (AVC).

On peut également estimer en chaque point la fraction d'anisotropie ou FA (*Fractionnai Anisotropy*), qui traduit l'écart à une diffusion isotrope en ce point. Plus précisément, la fraction d'anisotropie est définie par :

$$FA = \sqrt{\frac{3}{2}}\, \frac{\sqrt{\left(\lambda_1 - \overline{\lambda}\right)^2 + \left(\lambda_2 - \overline{\lambda}\right)^2 + \left(\lambda_3 - \overline{\lambda}\right)^2}}{\sqrt{\lambda_1^2 + \lambda_2^2 + \lambda_3^2}} \qquad\qquad (3)$$

On remarquera que la fraction d'anisotropie est comprise en la valeur 0 (diffusion isotrope) et la valeur 1 (diffusion selon une seule direction).

L'imagerie de diffusion, représentant la fraction anisotropique, permet de discriminer certaines régions du tissu cérébral et notamment les parties fibreuses, la diffusion des espèces ayant préférentiellement lieu le long des fibres.

En revanche, l'imagerie par résonance magnétique, conventionnelle ou de diffusion, ne permet pas d'estimer l'activité électrique cérébrale. On a pour ce faire recours à l'électroencéphalographie, l'électro-cortigraphie ou à la magnétoencéphalographie.

L'électroencéphalographie (EEG) consiste à mesurer l'activité électrique cérébrale au moyen d'électrodes placées sur le scalp du sujet. L'électrocortigraphie (ECoG) est plus invasive: elle consiste à mesurer directement l'activité électrique cérébrale au moyen d'électrodes placées directement sur la surface corticale. Enfin, la magnétoencéphalographie (MEG) consiste à mesurer indirectement l'activité électrique à partir des champs magnétiques engendrés par les courants dans le cerveau. Dans le cas de la MEG, les capteurs peuvent être des magnétomètres de précision (SQUIDs) capables de mesurer l'intensité du champ magnétiques et/ou des gradiomètres planaires capables de mesurer le gradient du champ magnétique dans un plan donné. Par exemple, chaque capteur peut comprendre trois capteurs élémentaires voire plus: un magnétomètre de précision mesurant l'intensité du champ magnétique en un point et deux gradiomètres planaires, perpendiculaires entre eux mesurant deux composantes du gradient du champ magnétique en ce point.

L'intérêt de l'imagerie MEG réside principalement dans le pré-diagnostic et le suivi thérapeutique de l'épilepsie. Toutefois, les images MEG ne sont pas facilement interprétables pour diagnostiquer ou pronostiquer certaines neuropathologies, notamment les tumeurs cérébrales, l'épilepsie, les accidents vasculaires cérébraux (AVC) la sclérose en plaques, la gliose ou la nécrose de certaines zones du tissu cérébral.

L'article de L. Hamid et al. intitulé « MEG-EEG fusion by Kalman filtering within a source analysis framework » publié dans Proc. of 35th International Conférence of the IEEE IMBS, Osaka, Japon, 3-7 Juillet 2013, pp. 4819-4822 décrit une méthode d'imagerie du tissu cérébral fondée sur la fusion de données MEG et de données EEG.

L'article de Bin He et al. intitulé « Multimodal functional neuroimaging : integrating functional MRI and EEG/MEG » publié dans IEEE Reviews in biomedical engineering, Vol. 1, 2008, pp. 23-40, décrit une méthode d'imagerie du tissu cérébral intégrant des images IRM fonctionnelles avec des données EEG/MEG.

L'article de C.J. Long et al. intitulé « Large scale Kalman filtering solutions to the electrophysical source localization problem - a MEG case study » publié dans Proc. of the 28th IEEE EMBS annual international conference, NYC, USA, Aug. 30-Sept. 3, 2006, pp. 4532-4535 décrit une méthode pour localiser l'activité cérébrale à partir de mesures MEG, utilisant un modèle dynamique pour les données EEG/MEG et un filtre de Kalman pour résoudre le problème d'inversion.

Le but de la présente invention est de proposer une nouvelle méthode d'imagerie du tissu cérébral qui facilite le diagnostic, le pronostic, ou le suivi thérapeutique d'un certain nombre des neuropathologies précitées.

## EXPOSÉ DE L'INVENTION

La présente invention est définie par une méthode d'imagerie par système de magnétoencéphalographie (MEG) dans laquelle :

(a) on détermine pour chaque voxel du tissu cérébral, des vecteurs propres et des valeurs propres d'une matrice de diffusion, au moyen d'un système d'IRM de diffusion;
(b) on calcule une matrice d'observation de l'état des courants électriques dans les différents voxels, à partir d'images du tissu cérébral fournies par un système d'IRM anatomique, d'un modèle de propagation électromagnétique et des positions des capteurs du système MEG;

(c) on calcule une matrice de transition d'état d'un modèle spatio-temporel particulaire donnant l'évolution d'un vecteur d'état représentatif des courants électriques dans les différents voxels, la matrice de transition d'état étant calculée à partir des vecteurs et valeurs propres de diffusion déterminés à l'étape (b) ainsi qu'à partir de paramètres électriques relatifs à chaque voxel, les paramètres électriques des différents voxels formant une particule du modèle spatio-temporel particulaire ;

(d) on estime au moyen d'un filtre de Kalman le vecteur d'état en un instant donné, à partir du vecteur d'état estimé en un instant précédent et d'un vecteur d'observation, représentatif des signaux physiologiques mesurés par les capteurs dudit système MEG au dit instant donné;

(e) on calcule la matrice de covariance de l'erreur *a posteriori* à partir du vecteur d'état estimé audit instant donné et du vecteur d'observation en cet instant;

les étapes (c),(d),(e) étant effectuées pour une pluralité de particules du modèle spatio-temporel particulaire ;

(f) on met à jour les poids des différentes particules en fonction des matrices de covariance de l'erreur *a posteriori* calculées à l'étape (e), le poids d'une particule étant d'autant plus augmenté que la covariance de l'erreur correspondante est faible ;

(g) on sélectionne la particule de plus fort poids parmi ladite pluralité de particules ;

(h) on représente sous forme d'image, au moins l'un des paramètres électriques de la particule de plus fort poids, pour les différents voxels du tissu cérébral.

Les paramètres électriques relatifs à un voxel $v$ comprennent avantageusement un paramètre de perte d'anisotropie, $\eta_v$, traduisant la perte d'anisotropie de propagation des courants électriques par rapport à l'anisotropie de diffusion, un paramètre de connectivité, $\kappa_v$, traduisant le degré de dépendance du courant électrique du voxel v par rapport aux voxels voisins, et un paramètre d'amplification ou d'atténuation spontanée, $k_{vv}$, traduisant l'amplification ou l'atténuation spontanée de courant électrique dans le voxel.

Les éléments diagonaux de la matrice de changement d'état sont avantageusement obtenus à partir des paramètres d'amplification ou d'atténuation spontanée des différents voxels.

De préférence, un élément de la matrice de changement d'état, $k_{vi}$, relatif à un couple de voxels voisins v,i est déterminé en modifiant les valeurs propres $(a_v, b_v, c_v)$ de la matrice de diffusion à l'aide du facteur de perte d'anisotropie $\eta_v$ pour obtenir un ellipsoïde de diffusion modifié, puis en appliquant un facteur d'homothétie égal au facteur de connectivité, $\kappa_v$, à l'ellipsoïde de diffusion ainsi modifié, et en recherchant le volume d'intersection de l'ellipsoïde homothétique $\left( E_v^{''} \right)$ ainsi obtenu avec le volume $(\Omega_i)$ dudit voxel voisin.

Le volume d'intersection dudit ellipsoïde homothétique avec le volume du voxel voisin peut être obtenu à l'aide d'une méthode de Monte Carlo.

La matrice de covariance de l'erreur *a posteriori* $\mathbf{P}_{t|t}^{\Pi}$ audit instant donné peut être obtenue par

$$\mathbf{P}_{t|t}^{\Pi} = \mathbf{P}_{t|t-1}^{\Pi} - \mathbf{K}_t^{\Pi} \mathbf{H} \mathbf{P}_{t|t-1}^{\Pi}$$ où $\mathbf{P}_{t|t-1}^{\Pi}$ est la matrice de covariance de l'erreur *a priori* audit instant donné, $\mathbf{K}_t^{\Pi}$ est le gain du filtre de Kalman et **H** est la matrice d'observation.

Les poids des différentes particules $\Pi_\ell$, $\ell = 1,...,L$, peuvent être mis à jour à l'aide d'une relation du type

$$\omega_\ell(t) \propto \omega_\ell(t-1) \left[ \mathrm{Tr} \left( \mathbf{P}_{t|t}^{\ell} \right) \right]^{-1}$$ où $\omega_\ell(t)$ est le poids de la particule $\ell$ audit instant donné, $\omega_\ell,(t\text{-}1)$ est le poids de cette même particule à l'instant précédent, $\mathbf{P}_{t|t}^{\ell}$ est la matrice de covariance de l'erreur *a posteriori* associée à la particule $\ell$, et Tr(.) est la fonction trace.

De préférence, lorsque le nombre de particules présentant un poids inférieur à un poids minimal prédéterminé est inférieur à un seuil prédéterminé, on procède à un rééchantillonnage d'importance de ces particules.

Le vecteur d'état du modèle spatio-temporel est avantageusement initialisé au moyen de $\hat{\mathbf{X}}_{0|0} = \mathbf{H}^T (\mathbf{H}\mathbf{H}^T + \lambda^{-1}\mathbf{I}_{N_c})^{-1}\mathbf{Y}_0$ où $\hat{\mathbf{X}}_{0|0}$ est la valeur initiale du vecteur d'état, $\mathbf{Y}_0$ est un vecteur d'observation initial, **H** est la matrice d'observation, $\mathbf{I}_{N_c}$ est la matrice identité de taille $N_c \times N_c$ et $\lambda$ est un paramètre de régularisation prédéterminé.

Ladite image d'au moins l'un des paramètres électriques peut être avantageusement combinée avec une image fournie par le système d'IRM anatomique.

## BRÈVE DESCRIPTION DES DESSINS

D'autres caractéristiques et avantages de l'invention apparaîtront à la lecture d'un mode de réalisation préférentiel de l'invention fait en référence aux figures jointes parmi lesquelles :

La Fig. 1 illustre de manière schématique le passage d'un modèle de diffusion IRMD à un modèle de diffusion des potentiels d'action dans le cerveau ;

La Fig. 2 illustre de manière schématique une méthode d'imagerie MEG assistée par IRMD selon un mode de réalisation de l'invention.

## EXPOSÉ DÉTAILLÉ DE MODES DE RÉALISATION PARTICULIERS

On considérera par la suite un système d'imagerie du tissu cérébral comprenant un système magnétoencéphalographique (MEG), un système d'imagerie IRM anatomique (IRM) et un système d'imagerie IRM de diffusion (IRMD). On comprendra de l'introduction que les systèmes d'imagerie IRM et IRMD pourront en pratique être implémentés sur une seule et même machine fonctionnant selon deux modes opératoires distincts comme décrit dans la partie introductive.

Le système magnétoencéphalographique comprend, de manière connue, un « casque MEG » placé à quelques centimètres de la boite crânienne du sujet. Ce casque comprend une pluralité de capteurs situés en différents points, chaque capteur pouvant être constitué d'un ou de plusieurs capteurs élémentaires. Les capteurs élémentaires peuvent être des magnétomètres de précision et des gradiomètres planaires. Alternativement, ils peuvent être des gradiomètres radiaux tels que ceux décrits dans l'article de J. Vrba et al. intitulé « Signal processing in magnetoencephalography », Methods 25, 249-271 (2001). Les mouvements de la tête du sujet sont en outre enregistrés et compensés grâce à des bobines placées en des points fixes par rapport à la tête du sujet et générant un champ magnétique dans une bande de fréquence éloignée de celle du signal MEG.

Les signaux acquis par les différents capteurs en un instant t donné peuvent être représentés par un vecteur $\mathbf{Y}_t$ de taille $N_c$. De même, on désigne par $\mathbf{X}_t$ le vecteur de taille $N_v$, où $N_v$ est le nombre de voxels de la zone à imager, un vecteur représentatif des courants locaux dans les différents voxels au temps t. Par courant local dans un voxel, on entend l'intensité du dipôle représentatif des courants dans le voxel en question.

On a alors la relation matricielle suivante :

$$\mathbf{Y}_t = \mathbf{H}\mathbf{X}_t + \mathbf{B}_t \qquad (4)$$

où $\mathbf{H}$ est une matrice de taille $N_c \times N_v$, dite matrice de transmission ou d'observation (*lead field matrix*) et $\mathbf{B}$ est un vecteur aléatoire de bruit de taille $N_c$ représentant le bruit de mesure sur les différents capteurs. On supposera que ce vecteur de bruit est constitué d'échantillons de variables aléatoires gaussiennes, indépendantes et identiquement distribuées (i.i.d).

En pratique, la matrice $\mathbf{H}$ est obtenue par simulation à partir d'une modélisation par éléments frontières ou une modélisation par éléments finis, le maillage obtenu étant effectué sur la base de l'image IRM anatomique du tissu cérébral, les positions des capteurs du système MEG par rapport au cerveau étant préalablement définis.

Par ailleurs, le système d'imagerie IRMD permet de déterminer, pour chaque voxel, les vecteurs propres du tenseur local de diffusion, comme rappelé plus haut.

Plus précisément, le système d'imagerie IRMD fournit pour chaque voxel v, les vecteurs propres $\overline{\mathbf{a}}_v, \overline{\mathbf{b}}_v, \overline{\mathbf{c}}_v$ du tenseur de diffusion ainsi que les valeurs propres correspondantes $a_v, b_v, c_v$ ($\overline{\mathbf{a}}_v, \overline{\mathbf{b}}_v, \overline{\mathbf{c}}_v$) est un repère orthonormé et l'on note dans la suite $\mathbf{a}_v = a_v \overline{\mathbf{a}}_v$, $\mathbf{b}_v = b_v \overline{\mathbf{b}}_v$ et $\mathbf{c}_v = c_v \overline{\mathbf{c}}_v$ avec $a_v \geq b_v \geq c_v$. Autrement dit, la direction principale de diffusion est donnée par le vecteur $\mathbf{a}_v$ et sa norme donne le coefficient de diffusion dans cette direction. Les vecteurs $\mathbf{b}_v$ et $\mathbf{c}_v$ donnent les première et seconde directions perpendiculaires de diffusion.

L'idée à la base de l'invention est d'élaborer un modèle spatio-temporel d'évolution des courants électriques à partir des tenseurs locaux de diffusion fournis par le système d'imagerie IRMD. En effet, si l'eau diffuse préférentiellement dans une direction, on peut supposer que le tissu est localement composé majoritairement de fibres axonales (substance blanche) le long desquelles se propagent les potentiels électriques. *A contrario,* dans le système ventriculaire, la diffusion de liquide céphalo-rachidien est sensiblement isotrope et la propagation des potentiels électriques est également sensiblement isotrope.

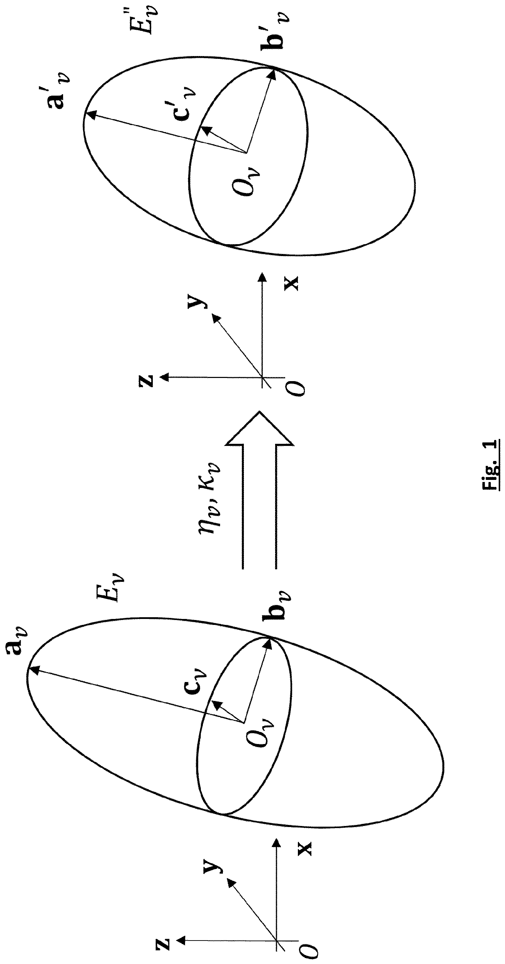

La Fig. 1 représente de manière schématique, pour un voxel v donné, le passage d'un modèle de diffusion IRMD à un modèle de propagation des potentiels électriques.

Sur la partie gauche de la figure, on a représenté l'ellipsoïde de diffusion $E_v$ relatif au voxel v, centré sur le point $O_v$ ainsi que ses vecteurs propres $\overline{\mathbf{a}}_v, \overline{\mathbf{b}}_v, \overline{\mathbf{c}}_v$ ($O, \overline{\mathbf{a}}_v, \overline{\mathbf{b}}_v, \overline{\mathbf{c}}_v$) est un repère orthonormé local. On note ($O, \mathbf{x}, \mathbf{y}, \mathbf{z}$) un repère orthonormé de référence du tissu cérébral et $M_v$ la matrice de passage du repère ($O, \mathbf{x}, \mathbf{y}, \mathbf{z}$) au repère ($O_v, \overline{\mathbf{a}}_v, \overline{\mathbf{b}}_v, \overline{\mathbf{c}}_v$).

On comprend que le voxel v est entouré de $3^3$-1 voxels voisins (non représentés).

Le modèle spatio-temporel d'évolution du courant électrique dans le voxel v est obtenu comme suit : L'ellipsoïde de diffusion $E_v$ est corrigé d'un facteur de perte d'anisotropie $\eta_v$, c'est-à-dire que celui-ci est déformé vers une forme sphérique. Ce facteur d'anisotropie traduit le fait que la directivité de propagation des potentiels électriques, ou de manière équivalente, du transport des charges électriques, est moindre que celle de la diffusion des molécules d'eau. La perte d'anisotropie peut être modélisée par une modification des valeurs propres $a_v, b_v, c_v$ sans modification des vecteurs propres $\overline{\mathbf{a}}_v, \overline{\mathbf{b}}_v, \overline{\mathbf{c}}_v$, soit :

$$a'_v = \left(1 - \eta_v\right)a_v + \eta_v$$

$$b'_v = \left(1 - \eta_v\right)b_v + \eta_v$$

$$c'_v = \left(1 - \eta_v\right)c_v + \eta_v \tag{5}$$

où $a'_v, b'_v, c'_v$ sont les valeurs propres modifiées. On voit notamment que pour le cas extrême où $\eta_v = 1$, l'ellipsoïde de diffusion est transformé en une sphère. Ce cas correspond à une perte d'anisotropie complète au niveau du voxel v .

Ainsi, à chaque voxel v est assigné un paramètre de perte d'anisotropie, $\eta_v$, qui caractérise la variation des valeurs propres $a'_v, b'_v, c'_v$ par rapport aux valeurs initiales $a_v, b_v, c_v$ mesurées par l'IRM de diffusion. L'utilité de ce paramètre de perte d'anisotropie sera expliquée par la suite.

Cette modification des valeurs propres en fonction du facteur d'anisotropie est effectuée pour chaque voxel v de l'image. On considère ensuite l'homothétique, d'un facteur d'homothétie, $\kappa_v$, de l'ellipsoïde de diffusion ainsi modifié, selon les trois vecteurs $\overline{\mathbf{a}}_v, \overline{\mathbf{b}}_v, \overline{\mathbf{c}}_v$. On détermine ensuite les intersections respectives entre l'homothétique, $E''_v$, de l'ellipsoïde modifié, $E'_v$ avec chacun des 26 voxels voisins, $\Omega_i$. On notera $\rho_{vi}$ le volume de $E''_v \cap \Omega_i$. Le coefficient de propagation entre le voxel i et le voxel v est donné par :

$$k_{vi} = \frac{\rho_{vi}}{\sum\limits_{j \in V(v)} \rho_{vj}} \tag{6}$$

où V(v) représente l'ensemble des voxels voisins du voxel $v$. Le facteur homothétique $\kappa_v$, tel que $0 \leq \kappa_v \leq \kappa_v^{\max}$, est en fait un paramètre de connectivité entre voxels qui représente la dépendance du courant du voxel v vis-à-vis de ses voisins, autrement la conductivité entre les voxels i et $v$. Ainsi, on comprendra que lorsque $\kappa_v = 0$, le courant du voxel v est indépendant de celui de ses voisins (voxel isolé électriquement), et lorsque $\kappa_v = \kappa_v^{\max}$ la connectivité avec ses voisins est maximale.

En pratique, il serait relativement complexe de calculer analytiquement les volumes des intersections d'un ellipsoïde avec les voxels voisins. Pour ce faire, on préfère recourir à une méthode de Monte-Carlo. Plus précisément, si l'on note $R_N$ une variable aléatoire ayant une loi normale centrée réduite, on peut générer un grand nombre de points à l'intérieur de $E''_v$ partirage de la variable aléatoire en question, les points générés ayant pour coordonnées $(a'R_N, b'R_N, c'R_N).\kappa_v$ dans le repère local $(O_v, \overline{\mathbf{a}}_v, \overline{\mathbf{b}}_v, \overline{\mathbf{c}}_v)$. Les coordonnées de ces points sont ensuite calculées dans le repère $(O, \mathbf{x}, \mathbf{y}, \mathbf{z})$, au moyen de la matrice de passage $\mathbf{M}_v$. Pour chaque voxel voisin i, on détermine ensuite la proportion des points générés qui tombent dans chacun des voxels voisins. Pour un nombre de points générés suffisamment grand, ces proportions donnent une estimation satisfaisante des coefficients $\rho_{vi}$.

En définitive, si l'on note $x_{v,t}$ le courant au point $O_v$ et au temps $t$, son évolution au temps $t + 1$ peut être modélisée par :

$$x_{v,t+1} = k_{vv}x_{v,t} + \sum_{i=1}^{26} k_{vi}x_{i,t} \tag{7}$$

Le nombre de voisins considéré est ici égal à 26, mais, le cas échéant, ce nombre peut être adapté à la configuration de voisinage. On comprend que le coefficient $k_{vv}$ n'est pas un coefficient de connectivité mais traduit l'amplification ou l'atténuation spontanée de courant dans le voxel $v$. En règle générale, on pourra considérer que le coefficient $k_{vv}$ est tel que $1\text{-}\varepsilon < k_{vv} < 1 + \varepsilon$ où $\varepsilon \square 1$. Il convient de remarquer que le fait que $k_{vv}$ puisse être supérieur à 1, ne signifie pas que le courant s'accroisse indéfiniment mais que celui-ci suit une loi exponentielle dans la fenêtre d'observation.

Si l'on note $\mathbf{X}_t$ le vecteur-colonne de taille $N_v$ dont les éléments sont les courants $x_{v,t}$, la relation (4) peut s'exprimer sous la forme :

$$\mathbf{X}_{t+1} = \mathbf{AX}_t + \mathbf{C} \tag{8}$$

où $\mathbf{A}$ est une matrice de transition spatio-temporelle de taille $N_v \times N_v$ dont les éléments sont les coefficients de connectivité précités et dont la diagonale est constituée des coefficients d'amplification ou d'atténuation spontanée $k_{vv}$. On remarque que l'on a introduit dans l'expression (7) un vecteur de bruit $\mathbf{C}$ de taille $N_v$ représentant le bruit du processus. Le vecteur de bruit $\mathbf{C}$ est constitué d'échantillons de variables aléatoires gaussiennes, indépendantes et identiquement distribuées. Les vecteurs de bruits $\mathbf{C}$ et $\mathbf{B}$ sont en outre considérés comme indépendants et leurs matrices de covariance sont respectivement notées $\mathbf{Q}$ et $\mathbf{R}$. Chaque courant $x_{v,t}$ correspond à l'intensité du courant dans le voxel v selon la direction principale définie par le vecteur $\mathbf{a}_v$.

Les équations (4) et (8) forment un modèle spatio-temporel d'évolution d'un vecteur d'état, repris ci-dessous :

$$\mathbf{X}_{t+1} = \mathbf{AX}_t + \mathbf{C} \tag{9-1}$$

$$\mathbf{Y}_t = \mathbf{HX}_t + \mathbf{B} \tag{9-2}$$

L'équation (9-1) représente l'évolution du vecteur d'état (caché) du modèle et l'équation (9-2) représente une mesure fournissant des observations de cet état. On peut considérer ce modèle comme un modèle de Markov caché où les états cachés forment une chaine de Markov.

L'estimation du vecteur d'état $\mathbf{X}_t$ est obtenue à l'aide d'une méthode de Monte Carlo séquentielle et plus précisément, à l'aide d'une méthode particulaire dans laquelle chaque particule est associée à un filtre de Kalman.

Chaque particule est en fait définie par un triplet $\Pi = (\eta, \kappa, \mathbf{k})$ où $\eta$ est un vecteur de taille $N_v$ dont les éléments sont les paramètres de perte d'anisotropie des différents voxels, $\kappa$ est un vecteur de de taille $N_v$ dont les éléments sont les paramètres de connectivité des différents voxels, et $\mathbf{k}$ est également un vecteur de taille $N_v$ dont les éléments sont les paramètres d'amplification ou d'atténuation spontanée $k_{vv}$ relatifs aux différents voxels.

A partir d'une particule $\Pi$ donnée, on peut déduire tous les éléments de la matrice $\mathbf{A}$. Pour traduire cette dépendance, la matrice d'évolution d'état est notée dans la suite $\mathbf{A}^\Pi$. Le modèle spatio-temporel d'évolution du vecteur d'état devient un modèle particulaire, représenté par :

$$\mathbf{X}_{t+1}^{\Pi(t)} = \mathbf{A}^{\Pi(t)} \mathbf{X}_t^{\Pi(t)} + \mathbf{C} \tag{10-1}$$

$$\mathbf{Y}_t = \mathbf{HX}_t^{\Pi(t)} + \mathbf{B} \tag{10-2}$$

où $\Pi(t)$ est une particule parmi une pluralité de L particules possibles, considérée à l'instant $t$. Dans la suite on pourra, selon le cas, omettre le temps $t$ ou au contraire utiliser un indice $\ell = 1,...,L$ pour indexer les particules, selon le contexte des explications. On comprendra dans tous les cas qu'il s'agit d'une population de L particules évoluant dans le temps, et qu'à chaque particule correspond un modèle spatio-temporel au sens des relations (10-1) et (10-2).

A chaque instant t, on effectue une prédiction du vecteur d'état à partir d'une estimation du vecteur d'état à l'instant précédent $t$-1. Cette prédiction (ou estimation $a$ $priori$) est faite pour chacune des particules $\Pi$, soit :

$$\hat{\mathbf{X}}_{t|t-1}^{\Pi(t)} = \mathbf{A}^{\Pi(t)} \hat{\mathbf{X}}_{t-1|t-1}^{\Pi(t-1)} \tag{11}$$

où $\hat{\mathbf{X}}_{t|t-1}^{\Pi(t)}$ est une prédiction du vecteur d'état à l'instant t à partir d'une estimation du vecteur d'état à l'instant $t$ - 1, obtenue sur la base d'une particule $\Pi(t$ -1).

Pour chaque particule $\Pi(t)$, on estime ensuite le vecteur d'état à l'instant t, compte tenu de l'observation $\mathbf{Y}_t$ par :

$$\hat{\mathbf{X}}_{t|t}^{\Pi(t)} = \hat{\mathbf{X}}_{t|t-1}^{\Pi(t)} + \mathbf{K}_t^{\Pi(t)} \left( \mathbf{Y}_t - \mathbf{H}\hat{\mathbf{X}}_{t|t-1}^{\Pi(t)} \right) \tag{12}$$

où $\hat{\mathbf{X}}_{t|t}^{\Pi(t)}$ est l'estimation *a posteriori* du vecteur d'état et $\mathbf{K}_t^{\Pi(t)}$ est le gain du filtre de Kalman associé à la particule $\Pi(t)$. Ainsi, de manière classique, l'estimation *a posteriori* du vecteur d'état est obtenue à partir de son estimation *a priori* et d'une mise à jour en fonction de l'écart entre l'observation et l'observation prédite.

Le gain de Kalman $\mathbf{K}_t^{\Pi(t)}$ est donné par les formules de récurrence :

$$\mathbf{K}_t^{\Pi(t)} = \mathbf{P}_{t|t-1}^{\Pi(t)}\mathbf{H}^T \left( \mathbf{H}\mathbf{P}_{t|t-1}^{\Pi(t)}\mathbf{H}^T + \mathbf{R} \right)^{-1} \tag{13-1}$$

$$\mathbf{P}_{t|t-1}^{\Pi(t)} = \mathbf{A}^{\Pi(t)}\mathbf{P}_{t-1|t-1}^{\Pi(t)} \left( \mathbf{A}^{\Pi(t)} \right)^T + \mathbf{Q} \tag{13-2}$$

$$\mathbf{P}_{t|t}^{\Pi(t)} = \mathbf{P}_{t|t-1}^{\Pi(t)} - \mathbf{K}_t^{\Pi(t)}\mathbf{H}\mathbf{P}_{t|t-1}^{\Pi(t)} \tag{13-3}$$

dans lesquelles $\mathbf{R}$ et $\mathbf{Q}$ représentent respectivement, comme déjà indiqué, la matrice de covariance du bruit de mesure et la matrice de covariance du bruit du processus, la matrice $\mathbf{P}_{t|t-1}^{\Pi(t)}$ et $\mathbf{P}_{t|t}^{\Pi(t)}$ représentent respectivement la matrice de covariance de l'erreur *a priori* et la matrice de covariance de l'erreur *a posteriori* autrement dit :

$$\mathbf{P}_{t|t-1}^{\Pi(t)} = E\left( \left( \mathbf{X}_t - \hat{\mathbf{X}}_{t|t-1}^{\Pi(t)} \right)\left( \mathbf{X}_t - \hat{\mathbf{X}}_{t|t-1}^{\Pi(t)} \right)^T \right) \tag{14}$$

$$\mathbf{P}_{t|t}^{\Pi(t)} = E\left( \left( \mathbf{X}_t - \hat{\mathbf{X}}_{t|t}^{\Pi(t)} \right)\left( \mathbf{X}_t - \hat{\mathbf{X}}_{t|t}^{\Pi(t)} \right)^T \right) \tag{15}$$

L'algorithme est initialisé à partir d'une estimation $\hat{\mathbf{X}}_{0|0}$ du vecteur d'état et une matrice de covariance *a priori* $\mathbf{P}_{0|0}$ du bruit du processus. Cette initialisation est la même pour toutes les particules.

Le vecteur $\hat{\mathbf{X}}_{0|0}$ peut être par exemple obtenu à partir de la résolution du problème inverse, c'est-à-dire en choisissant $\hat{\mathbf{X}}_{0|0} = \mathbf{X}_0$ où $\mathbf{X}_0$ vérifie (9-2):

$$\mathbf{Y}_0 = \mathbf{H}\mathbf{X}_0 + \mathbf{B} \tag{16}$$

Par ailleurs, la matrice $\mathbf{R}$ est déterminée à partir des mesures MEG sur tout ou partie de la durée de l'expérience. La matrice $\mathbf{Q}$ est déterminée à partir de la covariance des sources de chaque voxel après reconstruction, à partir des données MEG et IRM anatomique, et de la matrice $\mathbf{H}$ d'observation.

Différentes résolutions du problème inverse ont été proposées dans la littérature. On pourra notamment opter pour la méthode MNE *(Minimum Norm Estimate)* ou la méthode dSPM *(dynamic Statistical Parameter Mapping)* ou encore la méthode dite LORETA *(Low Resolution Electromagnetic Tomography)*. Ces différentes méthodes ont été passées en revue dans l'article de O. Hauk et al. intitulé « Comparison of noise-normalized minimum estimates of MEG analysis using multiple resolution metrics », parue dans Neuroimage, Vol. 54, 2011, pp. 1966-1974.

La solution MNE est donnée par :

$$\hat{\mathbf{X}}_{0|0} = \mathbf{H}^T \left( \mathbf{H}\mathbf{H}^T + \lambda^{-1}\mathbf{I}_{N_c} \right)^{-1} \mathbf{Y}_0 \qquad (17)$$

Où $\mathbf{I}_{N_c}$ est la matrice unité de taille $N_c \times N_c$ et $\lambda$ est un paramètre de régularisation qui peut être pris égal au rapport signal sur bruit.

A chaque instant t, on calcule les poids respectifs d'une pluralité de particules $\Pi$ indexées par $\ell = 1,.., L$, soit $\Pi_{\ell},(t)$, à partir des matrices de covariance d'erreur *a posteriori* $\mathbf{P}_{t|t}^{\ell}$. Plus précisément, on détermine les poids respectifs, $\omega_{\ell}(t)$ de ces particules au moyen de:

$$\sum_{\ell=1}^{L} \omega_{\ell}(t) = 1 \qquad (18)$$

et

$$\omega_{\ell}(t) \propto \omega_{\ell}(t-1)\left[ \mathrm{Tr}\left( \mathbf{P}_{t|t}^{\ell} \right) \right]^{-1} \qquad (19)$$

On comprendra de l'expression (19) que le poids d'une particule est mis à jour à partir de la matrice de covariance de l'erreur *a posteriori,* son poids s'accroissant d'autant plus que l'observation au temps t corrobore la prédiction et, réciproquement, son poids diminuant d'autant plus que l'observation au temps t s'en écarte.

Les poids $\omega_{\ell}(t)$ suivent une densité, dite densité d'importance *(importance density)* proportionnelle à la densité de probabilité *a posteriori* de $\Pi(t)$ :

$$p(\Pi(t)|\mathbf{Y}_{1:t}) \approx \sum_{\ell=1}^{L} \omega_{\ell}(t)\delta\left( \Pi(t) - \Pi_{\ell}(t) \right) \qquad (20)$$

où $\delta(.)$ est la distribution de Dirac.

Les particules sont initialement choisies équidistribuées dans un intervalle prédéterminé. Plus précisément, on pourra choisir à l'instant initial une même équidistribution des paramètres électriques $\eta_v, \kappa_v, k_v$ au sein d'intervalles respectifs, $[\eta^{\min}, \eta^{\max}], [\kappa^{\min}, \kappa^{\max}]$ et $[k^{\min}, k^{\max}]$ pour les différents voxels.

Afin d'éviter le phénomène de dégénérescence des particules (quasi-totalité des particules présentant un poids négligeable), on effectue, de manière connue en soi, un ré-échantillonnage d'importance à chaque fois que le nombre « efficace » de particules devient inférieur à un certain seuil. Ce ré-échantillonnage consiste à générer un nouvel ensemble de particules en les concentrant autour des particules présentant les poids les plus élevés.

Au terme de chaque fenêtre temporelle, on détermine alors la particule $\Pi^{opt} = (\eta^{opt}, \kappa^{opt}, \mathbf{k}^{opt})$ présentant le plus grand poids et on en déduit les valeurs, $\eta_v^{opt}, \kappa_v^{opt}, k_v^{opt}$ de chacun des voxels, $v = 1,.., N_v$.

Ces valeurs peuvent alors être affichées, isolément ou en combinaison, sous forme d'images. Les images ainsi générées pourront en outre être combinées à une image du système IRM anatomique. L'image en $k_v^{opt}$ traduit l'activité électrique spontanée du cerveau, l'image en $\kappa_v^{opt}$ représente sa connectivité électrique et l'image en $\eta_v^{opt}$ représente la perte d'anisotropie de sa conduction électrique.

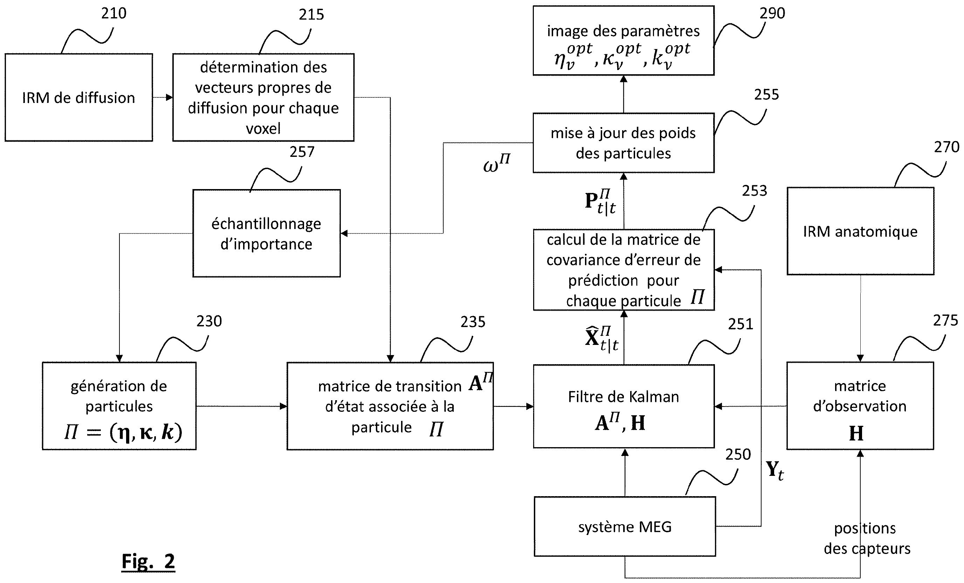

La Fig. 2 représente de manière schématique une méthode d'imagerie MEG assistée par IRMD selon un mode de réalisation de l'invention.

On a représenté sur la figure un système d'imagerie IRM de diffusion, 210, un système d'imagerie IRM anatomique, 270, et un système d'imagerie MEG, 250.

A partir, des images IRM de diffusion, on détermine en 215 les vecteurs propres, $\overline{\mathbf{a}}_v, \overline{\mathbf{b}}_v, \overline{\mathbf{c}}_v$, et les valeurs propres de diffusion, $a_v, b_v, c_v$ pour chaque voxel v .

Par ailleurs, on génère en 230 des particules $\Pi = (\eta, \kappa, \mathbf{k})$ au sens défini plus haut. Les particules sont générées, par exemple de manière équidistribuée (ou gaussienne centrée voire encore selon une autre loi si celle-ci connue *a priori),* lors de l'initialisation (au début de la fenêtre temporelle), puis sous contrôle d'un rééchantillonnage d'importance en cas de dégénérescence.

A chaque particule $\Pi$, on associe en 235 une matrice de transition $\mathbf{A}^\Pi$ du modèle d'état à partir des vecteurs/valeurs propres déterminés en 215. Plus précisément, les termes diagonaux de la matrice $\mathbf{A}^\Pi$ sont donnés par les éléments du vecteur $\mathbf{k}$. Les termes non diagonaux de la matrice $\mathbf{A}^\Pi$ sont obtenus pour un voxel v et ses voxels voisins $i \in V(v)$ en calculant les valeurs propres modifiées $a'_v, b'_v, c'_v$ à partir du facteur $\eta_v$ de perte d'anisotropie, en déterminant, au moyen de $\kappa_v$, l'homothétique $E''_v$ de l'ellipsoïde modifié et en en déduisant les coefficients $k_{vi}, i \in V(_v)$, par une méthode de Monte Carlo comme expliqué plus haut.

En outre, à partir d'images obtenues par le système d'imagerie anatomique 270 et des données de localisation des capteurs du système MEG (250) par rapport à un repérage anatomique du patient, on détermine en 275 la matrice de transmission $\mathbf{H}$. Ces images fournissent en effet une représentation 3D du cerveau permettant de le modéliser par éléments frontière ou par éléments finis. A l'aide d'un modèle de propagation électromagnétique dans le tissu cérébral et des positions des différents capteurs du système MEG, on peut alors déterminer la matrice de transmission $\mathbf{H}$.

Pour chaque particule $\Pi$, la matrice de transmission, $\mathbf{H}$, d'une part, et la matrice $\mathbf{A}^\Pi$ forment un modèle spatio-temporel d'évolution particulaire. Un filtrage de Kalman, 251, associé à la particule $\Pi$ permet d'estimer le vecteur d'état $\mathbf{X}^\Pi$ à l'instant t considéré,

compte tenu de la dernière observation $\mathbf{Y}_t$ fournie par le système MEG, 250.

Pour chaque particule, la trace de la matrice de covariance de l'erreur de prédiction *a posteriori* est déterminée en 253.

En 255, on met à jour les poids des différentes particules en fonction de la trace des différentes matrices de covariance, calculées en 253.

En fonction des poids des particules ainsi mis à jour, on procède le cas échéant (dégénérescence de l'ensemble des particules), à un rééchantillonnage d'importance, 257, pour générer un nouvel ensemble de L particules en 230. Par exemple, si le nombre de particules présentant un poids inférieur à un poids minimal prédéterminé excède un seuil donné, on peut procéder à un rééchantillonnage d'importance. Ce rééchantillonnage d'importance est caractérisé par le fait qu'il est d'autant plus serré que le poids de la particule est plus élevé.

La particule de plus fort poids, $\Pi^{opt}$, est déterminée, à chaque mise à jour des L particules, en 255. On peut alors afficher en 290, une image de l'amplification ou atténuation spontanée du courant électrique du cerveau à partir des $k_v^{opt}$, $v = 1,...,N_v$, une image de la connectivité électrique du cerveau à partir des $\kappa_v^{opt}$, $v = 1,..., N_v$, une image de la perte d'anisotropie de la conduction électrique par rapport à la direction préférentielle de diffusion des molécules d'eau à partir des $\eta_v^{opt}$, $v = 1,..., N_v$. Le cas échéant, les différentes images peuvent être combinées entre elles (représentation par couleur et intensité par exemple). Avantageusement, l'image du paramètre électrique est superposée ou combinée avec l'image IRM anatomique fournie par le système 270.

Ainsi, l'invention permet de déterminer la distribution spatiale des paramètres de perte d'anisotropie et/ou les paramètres de connectivité et/ou d'atténuation/amplification spontanée. De tels paramètres, conditionnant la conductivité de chaque voxel et sa relation avec son voisinage, et constituent, ensemble ou séparément, des biomarqueurs applicables à des fins diagnostiques, pronostiques, thérapeutiques et de suivi de l'évolution de l'état d'un patient.

Par exemple, une nécrose ou une gliose peut se traduire par une diminution du paramètre de connectivité. Par ailleurs, une augmentation du paramètre de perte d'anisotropie peut indiquer un oedème, envahissement tumoral ou une lésion axonale. Un paramètre d'amplification/atténuation supérieur à 1 peut être représentatif d'un foyer épileptogène.

## Revendications

1. Méthode d'imagerie par système de magnétoencéphalographie (MEG) **caractérisée en ce que** :

   (a) on détermine (215) pour chaque voxel du tissu cérébral, des vecteurs propres et des valeurs propres d'une matrice de diffusion, au moyen d'un système d'IRM de diffusion (210);
   (b) on calcule (275) une matrice d'observation **(H)** de l'état des courants électriques dans les différents voxels, à partir d'images du tissu cérébral fournies par un système d'IRM anatomique (270), d'un modèle de propagation électromagnétique et des positions des capteurs du système MEG;
   (c) on calcule (235) une matrice de transition d'état $(\mathbf{A}^\Pi)$ d'un modèle spatio-temporel particulier donnant l'évolution d'un vecteur d'état représentatif des courants électriques dans les différents voxels, la matrice de transition d'état $(\mathbf{A}^\Pi)$ étant calculée à partir des vecteurs et valeurs propres de diffusion déterminés à l'étape (b) ainsi qu'à partir de paramètres électriques relatifs à chaque voxel, les paramètres électriques des différents voxels formant une particule du modèle spatio-temporel particulier;

(d) on estime au moyen d'un filtre de Kalman (251) le vecteur d'état $(\hat{\mathbf{X}}_{t|t}^{\Pi})$ en un instant donné (*t*), à partir du vecteur d'état estimé en un instant précédent $(\hat{\mathbf{X}}_{t-1|t-1}^{\Pi})$ et d'un vecteur d'observation ($\mathbf{Y}_t$), représentatif des signaux physiologiques mesurés par les capteurs dudit système MEG au dit instant donné;

(e) on calcule la matrice de covariance de l'erreur *a posteriori* $(\mathbf{P}_{t|t}^{\Pi})$ à partir du vecteur d'état estimé audit instant donné et du vecteur d'observation en cet instant;

les étapes (c),(d),(e) étant effectuées pour une pluralité de particules du modèle spatio-temporel particulaire ;

(f) on met à jour les poids des différentes particules en fonction des matrices de covariance de l'erreur *a posteriori* calculées à l'étape (e), le poids d'une particule étant d'autant plus augmenté que la covariance de l'erreur correspondante est faible ;

(g) on sélectionne la particule de plus fort poids parmi ladite pluralité de particules ;

(h) on représente sous forme d'image, au moins l'un des paramètres électriques de la particule de plus fort poids, pour les différents voxels du tissu cérébral.

2. Méthode d'imagerie selon la revendication 1, **caractérisée en ce que** les paramètres électriques relatifs à un voxel v comprennent un paramètre de perte d'anisotropie, $\eta_v$, traduisant la perte d'anisotropie de propagation des courants électriques par rapport à l'anisotropie de diffusion, un paramètre de connectivité, $\kappa_v$, traduisant le degré de dépendance du courant électrique du voxel v par rapport aux voxels voisins, et un paramètre d'amplification ou d'atténuation spontanée, $k_{vv}$, traduisant l'amplification ou l'atténuation spontanée de courant électrique dans le voxel.

3. Méthode d'imagerie selon la revendication 2, **caractérisée en ce que** les éléments diagonaux de la matrice de changement d'état sont obtenus à partir des paramètres d'amplification ou d'atténuation spontanée des différents voxels.

4. Méthode d'imagerie selon la revendication 2, **caractérisée en ce qu'**un élément de la matrice de changement d'état, $k_{vi}$, relatif à un couple de voxels voisins v,i est déterminé en modifiant les valeurs propres ($a_v, b_v, c_v$) de la matrice de diffusion à l'aide du facteur de perte d'anisotropie $\eta_v$ pour obtenir un ellipsoïde de diffusion modifié, puis en appliquant un facteur d'homothétie égal au facteur de connectivité, $\kappa_v$, à l'ellipsoïde de diffusion ainsi modifié, et en recherchant le volume d'intersection de l'ellipsoïde homothétique ($E''_v$) ainsi obtenu avec le volume ($\Omega_i$) dudit voxel voisin.

5. Méthode d'imagerie selon la revendication 4, **caractérisée en ce que** le volume d'intersection dudit ellipsoïde homothétique avec le volume du voxel voisin est obtenu à l'aide d'une méthode de Monte Carlo.

6. Méthode d'imagerie selon l'une des revendications précédentes, **caractérisée en ce que** la matrice de covariance de l'erreur *a posteriori* $\mathbf{P}_{t|t}^{\Pi}$ audit instant donné est obtenue par $\mathbf{P}_{t|t}^{\Pi} = \mathbf{P}_{t|t-1}^{\Pi} - \mathbf{K}_t^{\Pi} \mathbf{H} \mathbf{P}_{t|t-1}^{\Pi}$ où $\mathbf{P}_{t|t-1}^{\Pi}$ est la matrice de covariance de l'erreur *a priori* audit instant donné, $\mathbf{K}_t^{\Pi}$ est le gain du filtre de Kalman et H est la matrice d'observation.

7. Méthode d'imagerie selon la revendication 6, **caractérisée en ce que** les poids des différentes particules $\Pi_\ell$, $\ell = 1,..., L$, sont mis à jour à l'aide d'une relation du type $\omega_\ell(t) \propto \omega_\ell(t-1) \left[ \mathrm{Tr}\left( \mathbf{P}_{t|t}^{\ell} \right) \right]^{-1}$ où $\omega_\ell(t)$ est le poids de la particule $\ell$ audit instant donné, $\omega_\ell(t-1)$ est le poids de cette même particule à l'instant précédent, $\mathbf{P}_{t|t}^{\ell}$ est la matrice de covariance de l'erreur *a posteriori* associée à la particule $\ell$, et Tr(.) est la fonction trace.

8. Méthode d'imagerie selon la revendication 7, **caractérisée en ce que**, lorsque le nombre de particules présentant un poids inférieur à un poids minimal prédéterminé est inférieur à un seuil prédéterminé, on procède à un rééchantillonnage d'importance de ces particules.

9. Méthode d'imagerie selon l'une des revendications précédentes, **caractérisée en ce que** le vecteur d'état du modèle spatio-temporel est initialisé au moyen de $\hat{\mathbf{X}}_{0|0} = \mathbf{H}^T (\mathbf{H}\mathbf{H}^T + \lambda^{-1}\mathbf{I}_{N_c})^{-1}\mathbf{Y}_0$ où $\hat{\mathbf{X}}_{0|0}$ est la valeur initiale du vecteur d'état,

$\mathbf{Y}_0$ est un vecteur d'observation initial, $\mathbf{H}$ est la matrice d'observation, $\mathbf{I}_{N_c}$ est la matrice identité de taille $N_c \times N_c$ et $\lambda$ est un paramètre de régularisation prédéterminé.

10. Méthode d'imagerie selon l'une des revendications précédentes, **caractérisée en ce que** la dite image d'au moins l'un des paramètres électriques est combinée avec une image fournie par le système d'IRM anatomique.

**Patentansprüche**

1. Verfahren zur Bildgebung über ein Magnetoenzephalographie-System (MEG), **dadurch gekennzeichnet, dass** es folgende Schritte umfasst:

(a) Bestimmen (215) von Eigenvektoren und Eigenwerten einer Diffusionsmatrix mittels eines Diffusions-MRT-Systems (210) für jedes Voxel des Hirngewebes;
(b) Berechnen (275) einer Beobachtungsmatrix (**H**) des Zustands der elektrischen Ströme in den verschiedenen Voxeln ausgehend von Bildern des Gehirngewebes, die von einem anatomischen MRT-System bereitgestellt werden (270), von einem Modell der elektromagnetischen Ausbreitung und den Positionen von Sensoren des MEG-Systems;
(c) Berechnen (235) einer Zustandsübergangsmatrix ($\mathbf{A}^{\Pi}$) eines räumlich-zeitlichen Teilchenmodells, die die Entwicklung eines Zustandsvektors angibt, der repräsentativ ist für die elektrischen Ströme in den verschiedenen Voxeln, wobei die Zustandsübergangsmatrix ($\mathbf{A}^{\Pi}$) ausgehend von den in Schritt (b) ermittelten Diffusionseigenvektoren und -werten sowie von elektrischen Parametern bezüglich jedes Voxels berechnet wird, wobei die elektrischen Parameter der verschiedenen Voxel ein Teilchen des räumlich-zeitlichen Teilchenmodells bilden;
(d) Schätzen des Zustandsvektors $\left(\widehat{X}_{t/t}^{\Pi}\right)$ zu einem gegebenen Zeitpunkt (t) mittels eines Kalman-Filters (251) ausgehend von dem zu einem vorherigen Zeitpunkt geschätzten Zustandsvektor $\left(\widehat{X}_{t-1/t-1}^{\Pi}\right)$ und einem Beobachtungsvektor (**Yt**), der repräsentativ ist für die physiologischen Signale, die von den Sensoren des MEG-Systems zu dem gegebenen Zeitpunkt gemessen werden;
(e) Berechnen der Kovarianzmatrix des a posteriori-Fehlers $\left(P_{t/t}^{\Pi}\right)$ ausgehend von dem zu dem gegebenen Zeitpunkt geschätzten Zustandsvektor und dem Beobachtungsvektor zu diesem Zeitpunkt;
wobei die Schritte (c), (d), (e) für eine Vielzahl von Teilchen des räumlich-zeitlichen Teilchenmodells durchgeführt werden;
(f) Aktualisieren der Gewichte der verschiedenen Teilchen in Abhängigkeit von den in Schritt (e) berechneten Kovarianzmatrices des a posteriori-Fehlers, wobei das Gewicht eines Teilchens umso mehr erhöht wird, als die Kovarianz des entsprechenden Fehlers schwach ist;
(g) Auswählen des Teilchens mit dem größten Gewicht aus der Vielzahl von Teilchen;
(h) Darstellen in Form eines Bildes von zumindest einem der elektrischen Parameter des Teilchens mit dem größten Gewicht für die verschiedenen Voxel des Hirngewebes.

2. Bildgebungsverfahren nach Anspruch 1, **dadurch gekennzeichnet, dass** die elektrischen Parameter zu einem Voxel v einen Anisotropie-Verlust-Parameter $\eta_v$ umfassen, der den Verlust an Anisotropie bei der Ausbreitung der elektrischen Ströme bezüglich der Diffusions-Anisotropie ausdrückt, einen Konnektivitätsparameter $\kappa_v$, der den Grad der Abhängigkeit des elektrischen Stroms des Voxels v in Bezug auf benachbarte Voxel ausdrückt, und einen Parameter spontaner Verstärkung bzw. Dämpfung, $k_{vv}$, welcher die spontane Verstärkung bzw. Dämpfung des elektrischen Stroms in dem Voxel ausdrückt.

3. Bildgebungsverfahren nach Anspruch 2, **dadurch gekennzeichnet, dass** die diagonalen Elemente der Zustandsänderungsmatrix ausgehend von den Parametern der spontanen Verstärkung bzw. Dämpfung der verschiedenen Voxel erhalten werden.

4. Bildgebungsverfahren nach Anspruch 2, **dadurch gekennzeichnet, dass** ein Element der Zustandsänderungsmatrix, $k_{vi}$, bezogen auf ein Paar von benachbarten Voxeln v,i dadurch bestimmt wird, dass die Eigenwerte ($a_v$, $b_v$, $c_v$) der Diffusionsmatrix mit Hilfe des Anisotropie-Verlustfaktors $\eta_v$ bestimmt wird, um ein modifiziertes Diffusions-Ellipsoid zu erhalten, dann ein Homothetiefaktorgleich dem Konnektivitätsfaktor $\kappa_v$ auf das so modifizierte Diffusions-Ellipsoid angewendet wird, und das Schnittvolumen des so erhaltenen homothetischen Ellipsoids $E''_v$ mit dem Volumen ($\Omega_i$) des benachbarten Voxels herausgefunden wird.

5. Bildgebungsverfahren nach Anspruch 4, **dadurch gekennzeichnet, dass** das Schnittvolumen des homothetischen Ellipsoids mit dem Volumen des benachbarten Voxels mittels einer Monte-Carlo-Methode erhalten wird.

6. Bildgebungsverfahren nach einem der vorangehenden Ansprüche, **dadurch gekennzeichnet, dass** die Kovarianzmatrix des a posteriori-Fehlers $P^{\Pi}_{t/t}$ zu dem gegebenen Zeitpunkt erhalten wird durch $P^{\Pi}_{t/t} = P^{\Pi}_{t/t-1} - K^{\Pi}_t H P^{\Pi}_{t/t-1}$, worin $P^{\Pi}_{t/t-1}$ die Kovarianzmatrix des a priori-Fehlers zu dem gegebenen Zeitpunkt ist, $K^{\Pi}_t$ die Verstärkung des Kalman-Filters ist und H die Beobachtungsmatrix ist.

7. Bildgebungsverfahren nach Anspruch 6, **dadurch gekennzeichnet, dass** die Gewichte der verschiedenen Teilchen $\Pi_\ell, \ell = 1,..., L$ mit Hilfe eines Verhältnisses der Art $\omega_\ell(t) \propto \omega_\ell(t-1)\left[ \mathrm{Tr}\left( \mathbf{P}^\ell_{t/t} \right) \right]^{-1}$ aktualisiert werden, wobei $\omega_\ell(t)$ das Gewicht des Teilchens $\ell$ zu dem gegebenen Zeitpunkt ist, $\omega_\ell(t-1)$ das Gewicht des gleichen Teilchens zu dem vorherigen Zeitpunkt ist, $\mathbf{P}^\ell_{t/t}$ die Kovarianzmatrix des dem Teilchen $\ell$ zugeordneten a posteriori-Fehlers ist und Tr(.) die Trace-Funktion ist.

8. Bildgebungsverfahren nach Anspruch 7, **dadurch gekennzeichnet, dass** dann, wenn die Anzahl von Teilchen mit einem geringeren Gewicht als ein vorbestimmtes Mindestgewicht kleiner ist als ein vorbestimmter Schwellenwert, eine Wichtigkeits-Neuabtastung dieser Teilchen ausgeführt wird.

9. Bildgebungsverfahren nach einem der vorangehenden Ansprüche, **dadurch gekennzeichnet, dass** der Zustandsvektor des räumlich-zeitlichen Modells mit $\hat{\mathbf{X}}_{0|0} = \mathbf{H}^T(\mathbf{HH}^T + \lambda^{-1}\mathbf{I}_{N_c})^{-1}\mathbf{Y}_0$ initialisiert, worin $\hat{\mathbf{X}}_{0/0}$ der Anfangswert des Zustandsvektors ist, $Y_o$ ein anfänglicher Beobachtungsvektor ist, **H** die Beobachtungsmatrix ist, $\mathbf{I}_{N_c}$ die Identitätsmatrix der Größe $N_c \times N_c$ und $\lambda$ ein vorbestimmter Regularisierungsparameter ist.

10. Bildgebungsverfahren nach einem der vorangehenden Ansprüche, **dadurch gekennzeichnet, dass** das Bild von mindestens einem der elektrischen Parameter mit einem Bild kombiniert wird, das von dem anatomischen MRI-System bereitgestellt wird.

**Claims**

1. A method for imaging by a magnetoencephalography (MEG) system **characterised in that**:

    (a) for each voxel of the brain tissue, eigenvectors and eigenvalues of a diffusion matrix are determined (215), by means of a diffusion MRI system (210);
    (b) an observation matrix (H) of the state of the electrical currents in the different voxels is calculated (275), from images of the brain tissue which are provided by an anatomical MRI system (270), of an electromagnetic propagation model and positions of the sensors of the MEG system;
    (c) a state transition matrix ($A^{II}$) of a particle spatio-temporal model giving the evolution of a state vector representative of the electrical currents in the different voxels is calculated (235), the state transition matrix ($A^{II}$) being calculated from the diffusion eigenvectors and eigenvalues determined in step (b) as well as from electrical parameters related to each voxel, the electrical parameters of the different voxels forming a particle of the particle spatio-temporal model;

    (d) by means of a Kalman filter (251), the state vector $\left(\hat{\mathbf{X}}^{\Pi}_{t|t}\right)$ is estimated at a given instant (t), from the state vector estimated at a previous instant $\left(\hat{\mathbf{X}}^{\Pi}_{t-1|t-1}\right)$ and an observation vector ($\mathbf{Y}_t$), representative of the physio-

logical signals measured by the sensors of said MEG system at said given instant;

(e) the *a posteriori* error covariance matrix $\left(\mathbf{P}_{t|t}^{\Pi}\right)$ is calculated from the state vector estimated at said given instant and the observation vector at this instant;

steps (c), (d), (e) being performed for a plurality of particles of the particle spatio-temporal model;

(f) the weights of the different particles are updated as a function of the *a posteriori* error covariance matrices calculated in step (e), the weight of a particle being all the more increased as the corresponding error covariance is low;

(g) the highest-weight particle is selected from said plurality of particles;

(h) at least one of the electrical parameters of the highest-weight particle is represented as an image, for the different voxels of the brain tissue.

2. The imaging method according to claim 1, **characterised in that** the electrical parameters related to a voxel v comprise an anisotropy loss parameter, $\eta_v$, reflecting the electrical current propagation anisotropy loss with respect to the diffusion anisotropy, a connectivity parameter, Kv, reflecting the dependence degree of the electrical current of the voxel v to the neighbouring voxels, and a spontaneous amplification or attenuation parameter, $k_w$, reflecting the spontaneous amplification or attenuation of the electrical current in the voxel.

3. The imaging method according to claim 2, **characterised in that** the diagonal elements of the state change matrix are obtained from the spontaneous amplification or attenuation parameters of the different voxels.

4. The imaging method according to claim 2, **characterised in that** an element of the state change matrix, $k_{vi}$, related to a couple of neighbouring voxels v,i is determined by modifying the eigenvalues $(o_v, b_v, c_v)$ of the diffusion matrix using the anisotropy loss factor $\eta_v$, to obtain a modified diffusion ellipsoid, and then by applying an homothety factor equal to the connectivity factor, $K_v$, to the diffusion ellipsoid thus modified, and by searching for the intersection volume of the homothetic ellipsoid $\left(E_v^*\right)_i$ thus obtained with the volume $(\Omega_i)$ of said neighbouring voxel.

5. The imaging method according to claim 4, **characterised in that** the intersection volume of said homothetic ellipsoid with the volume of the neighbouring voxel is obtained using a Monte Carlo method.

6. The imaging method according to one of the preceding claims, **characterised in that** the *a posteriori* error covariance matrix $\mathbf{P}_{t|t}^{\Pi}$ at said given instant is obtained by $\mathbf{P}_{t|t}^{\Pi} = \mathbf{P}_{t|t-1}^{\Pi} - \mathbf{K}_t^{\Pi}\mathbf{H}\mathbf{P}_{t|t-1}^{\Pi}$ where $\mathbf{P}_{t|t-1}^{\Pi}$ is the *a priori* error covariance matrix at said given instant, $\mathbf{K}_t^{\Pi}$ is the gain of the Kalman filter and $\mathbf{H}$ is the observation matrix.

7. The imaging method according to claim 6, **characterised in that** the weights of the different particles $\Pi_\ell$, $\ell = 1,...,$ L are updated using a relationship of the type $\omega_\ell(t) \propto \omega_\ell(t-1)\left[\mathrm{Tr}\left(\mathbf{P}_{t|t}^{\ell}\right)\right]^{-1}$ where $\omega_\ell(t)$ is the weight of the particle $\ell$ at said given instant, $\omega_\ell(t-1)$ is the weight of the same particle at the previous instant, $\mathbf{P}_{t|t}^{\ell}$ is the *a posteriori* error covariance matrix associated with the particle $\ell$, and Tr(.) is the trace function.

8. The imaging method according to claim 7, **characterised in that**, when the number of particles having a weight lower than a predetermined minimum weight is lower than a predetermined threshold, a significance resampling for these particles is performed.

9. The imaging method according to one of the preceding claims, **characterised in that** the state vector of the spatio-temporal model is initialised by means of $\hat{\mathbf{X}}_{0|0} = \mathbf{H}^T(\mathbf{H}\mathbf{H}^T + \lambda^{-1}\mathbf{I}_{N_c})^{-1}\mathbf{Y}_0$ where $\hat{\mathbf{X}}_{0|0}$ is the initial value of the state vector, $\mathbf{Y}_0$ is an initial observation vector, $\mathbf{H}$ is the observation matrix, $\mathbf{I}_{N_c}$ is the identity matrix having the size $N_c{\times}N_c$ and $\lambda$ is a predetermined regularisation parameter.

10. The imaging method according to one of the preceding claims, **characterised in that** said image of at least one of the electrical parameters is combined with an image provided by the anatomical MRI system.

EP 3 182 884 B1

**Fig. 1**

**Fig. 2**

EP 3 182 884 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **S. MORI et al.** Diffusion magnetic resonance imaging: its principle and applications. *The Anatomical Record,* 1999, vol. 257, 102-109
- MEG-EEG fusion by Kalman filtering within a source analysis framework. **L. HAMID et al.** Proc. of 35th International Conférence of the IEEE. IMBS, 03 Juillet 2013, 4819-4822
- **BIN HE et al.** Multimodal functional neuroimaging : integrating functional MRI and EEG/MEG. *IEEE Reviews in biomedical engineering,* 2008, vol. 1, 23-40
- Large scale Kalman filtering solutions to the electrophysical source localization problem - a MEG case study. **C.J. LONG et al.** Proc. of the 28th IEEE EMBS annual international conference. NYC, 30 Août 2006, 4532-4535
- **J. VRBA et al.** Signal processing in magnetoencephalography. *Methods,* 2001, vol. 25, 249-271
- **O. HAUK et al.** Comparison of noise-normalized minimum estimates of MEG analysis using multiple resolution metrics. *Neuroimage,* 2011, vol. 54, 1966-1974